# EUROPEAN PATENT SPECIFICATION

(11) **EP 2 163 551 B1**
(45) Date of publication and mention of the grant of the patent: **16.11.2011**
(21) Application number: 09011438.0
(22) Date of filing: 07.09.2009
(51) Int. Cl.: C07D 311/58

(54) **An improved process for the preparation of nebivolol hydrochloride**
Verbesserter Herstellungsprozess für Nebivolol-Hydrocholorid
Procédé amélioré pour la préparation d'hydrochlorure de nebivolol

(30) Priority: 08.09.2008 IN MU18882008
(43) Date of publication of application: 17.03.2010
(73) Proprietor: Cadila Pharmaceuticals Ltd., Ahmedabad 382 210 GUJ (IN)
(72) Inventor: Khamar, Bakulesh Mafatlal, Ahmedabad 382 210 GUJ (IN); Thakor, Indrajit, Ahmedabad 382 210 GUJ (IN); Patel, Mahesh, Ahmedabad 382 210 GUJ (IN); Jadav, Kalpesh, Ahmedabad 382 210 GUJ (IN); Parikh, Ankur, Ahmedabad 382 210 GUJ (IN); Joshi, Atul Chhotalal, Ahmedabad 382 210 GUJ (IN); Ponnaiah, Ravi, Ahmedabad 382 210 GUJ (IN); Modi, Indravadan Ambalal, Ahmedabad 382 210 GUJ (IN)
(74) Representative: Rigby, Barbara Nicole

(56) References cited:
- WO-A-2006/025070
- US-B1- 6 545 040
- BAI YIHUI ET AL: "A mild synthesis of .alpha.,.alpha.'-ÄiminobismethyleneÜbisÄ6- fluoro 3,4- dihydro-2H-1-benzopyran-2-methanolÜ" JOURNAL OF CHEMICAL RESEARCH. SYNOPSES, SCIENCE & TECHNOLOGY LETTERS, LONDON, GB, no. 12, 1 January 2006 (2006-01-01), pages 807-808, XP009097176 ISSN: 0308-2342
- CHANDRASEKHAR S ET AL: "Enantioselective Total Synthesis of the Antihypertensive Agent (S,R,R,R)-Nebivolol" TETRAHEDRON, ELSEVIER SCIENCE PUBLISHERS, AMSTERDAM, NL, vol. 56, no. 34, 18 August 2000 (2000-08-18), pages 6339-6344, XP004214993 ISSN: 0040-4020

## Description

### FIELD OF THE INVENTION:

The present invention relates to an improved, one pot synthesis of pharmaceutically active 2, 2'-iminobisethanol derivative i.e. (±)-[2R*[1S*,5S*(S*)]]-α,α¹-[iminobis(methylene)]bis[6-fluor0-3,4-dihydro-2H-1-benzo-pyran-2-methanol] or its pharmaceutically acceptable salts, more particularly hydrochloride saft

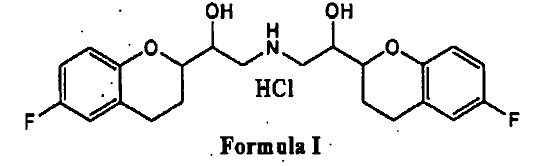

### BACKGROUND OF THE INVENTION:

US patent no. 4,654,362 disclosed 2, 2'-iminobisethanol derivatives which are antihypertensive agents. Among them Nebivolol, chemically known as (±)-[2R*[1S*,59*(S*)]]-α,α¹ -[minobis(methylene)]bis[6-fluoro-3,4-dihydro-2H-1-benzo-pyran-2-methanol] is the most important antihypertensive agent and it is represented by the following structural formula

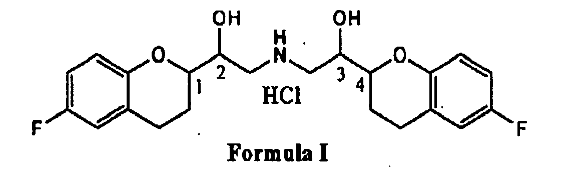

The above structure has four stereogenic centers, which are indicated with No. 1, 2, 3 and 4. Nebivolol is a mixture of equal amounts of 2 enantiomers having the SRRR- and the RSSS-configuration respectively

Methods for preparation of Nebivolol are disclosed in U.S. patent nos. 4,654,362, 6,545,040, 5,759,580; U.S. patent publication nos. 2007/259950, 2007/021623 and PCT publication WO20071083318.

US patent no. 4,654,362 describes a process for the conversion of 6-fluoro-3, 4-dihydro-2H-1-benzopyran-2-carboxaldehyde (II) into isomeric mixtures of 6-fluoro-3, 4-dihydro-2-oxiranyl-2H-1-benzopyran (III). The mixture of oxiranes represented by the formula (III) are separated by column chromatography to obtain A-isomer of (III) (i.e., III-A) from the first fraction and B-isomer of (III) (i.e., III-B) from the second fraction. The A-isomer of (III) is then treated with benzylamine to obtain the benzylated A-isomer of (III) which is reacted with the B-isomer in presence of oxalic acid to obtain the oxalate salt of benzylated Nebivolol. Furthermore the oxalate salt as obtained is treated with an alkali to obtain the free benzylated Nebivolol base (V), (Scheme-I).

The multiple steps in the process make it cumbersome and lead to increase in utilities and in the manufacturing time cycle of the active pharmaceutical ingredient. Moreover the purity of the benzylated Nebivolol base is relatively low.

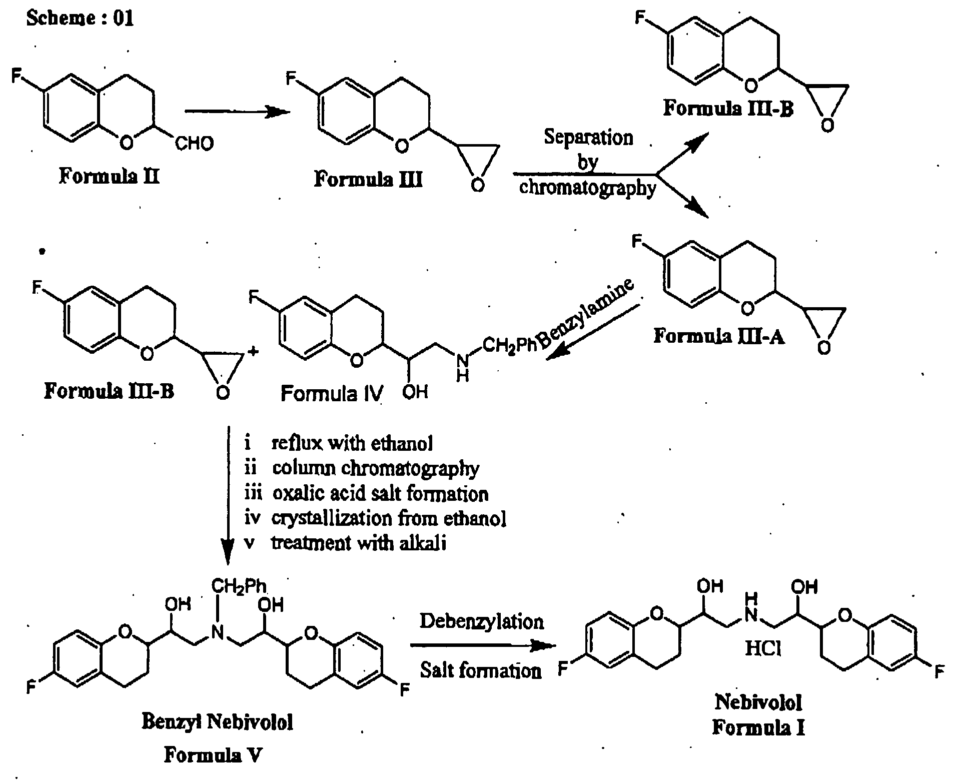

US patent no. 6,545,040 discloses a process for the preparation of specifically the RSSS and SRRR isomers of Nebivolol independently; the step-wise details are described below.
[1] 6-fluoro-chroman-2-carboxylic acid is resolved with (+)dehydroabietyl amine to give (+)-(S)-6-fluoro-3,4-dihydro-2H-1-benzopyran-2-carboxylic acid which is converted into (+)-(S)-6-fluoro-3,4-dihydro-2H-benzopyran-2-carboxaldehyde through multiple stage processes provides (+)-[S(S)]-6-fluoro-3,4-dihydro-2-oxyranyl-2H-1-benzopyran (intermediate-5).
[2] (-)-(R)-6-fluoro-3,4-dihydro-2H-1-benzopyran-2-carboxylic acid is converted to corresponding methyl ester. Further methyl ester compound after multiple stage processes provides (-)-[R(S)]6-fluoro-3,4-dihydro-2-oxyranyl-2H-1-benzopyran using column chromatographic separation which is then treated with benzyl amine to provide (-)-[R(S)]-6-fluoro-3,4-dihydro-α-[[(phenylmethyl)amino]methyl]-2H-1-benzopyran-2-methanol. (Intermediate-10). The crude is crystallized using acetonitrile to give pure intermediate 10 (38.1 % yield).
[3] Intermediate-5 and intermediate-10 are reacted in ethanol for 4 hours at reflux temperature to give [2R,αS,2'S,α'S]-α,α'[[(phenylmethyl)imino]bismethylene]bis[6-fluoro-3,4-dihydro-2H-1-benzopyran-2-methanol] (intermediate-11).
[4] Intermediate-11 is taken up in 2-methoxyethanol and hydrogenated using palladium on charcoal catalyst (10% w/w) to give a product which is isolated and purified using column chromatography followed by two crystallizations from acetonitrile to give 42% of [2R,αS,2'S,α'S]-α,α'-iminobismethylene]bis[6-fluoro-3,4-dihydro-2H-1-benzopyran-2-methanol: m.p. 142-7°C.

The said process for the independent preparation of RSSS and SRRR isomers of Nebivolol involves the use of reagents like di-isobutyl aluminium hydride (DIBAL) which is hazardous; (+)-1,2,3,4,4a,9,10,10a-octahydro-1,4a-dimethyl-7- (1-methylethyl-1-phenathrene methan-amine [(+)-dehydroabietylamine]. The process also involves a large number of steps thereby increasing the utilities, manpower and time required to complete the production cycle, rendering the process commercially expensive.

US patent no. 5,759,580 describes a process for preparing Nebivolol hydrochloride from a mixture containing the Nebivolol base with desired RSSS+SRRR isomers which is contaminated with undesired RSRR and SRSS diastereomers, using ethanol as both as reaction solvent and recrystallization solvent. The process involves the use of Nebivolol base as the starting material containing different isomeric impurities. Hence the end product results in a very low yield (6.6%) of the desired isomers having the SRRR- and the RSSS-configuration in Nebivolol hydrochloride.

U.S. patent publication No. 2007/259950 discloses process for preparation of Nebivolol and its pharmaceutically acceptable salts. In the synthetic steps (±)-[1S*(R*)]-6-fluoro-3,4-dihydro-α[[(phenylmethyl)amino]methyl]-2H-1-benzo pyran-2-methanol (compound of formula IV) is treated with (±)-[1S*(S)*]-6-fluoro-3,4-dihydro-2H-1-benzopyran (compound of formula III-B) in methanol and isolated at -10 to -15°C temperature after 10-15 hours of stirring to get desired isomer of benzyl Nebivolol (compound of formula V). Benzyl Nebivolol was then debenzylated with 10 % palladium on charcoal as catalyst for hydrogenation to give Nebivolol base, followed by treating with hydrochloric acid in an organic solvent to give Nebivolol hydrochloride (Scheme II).

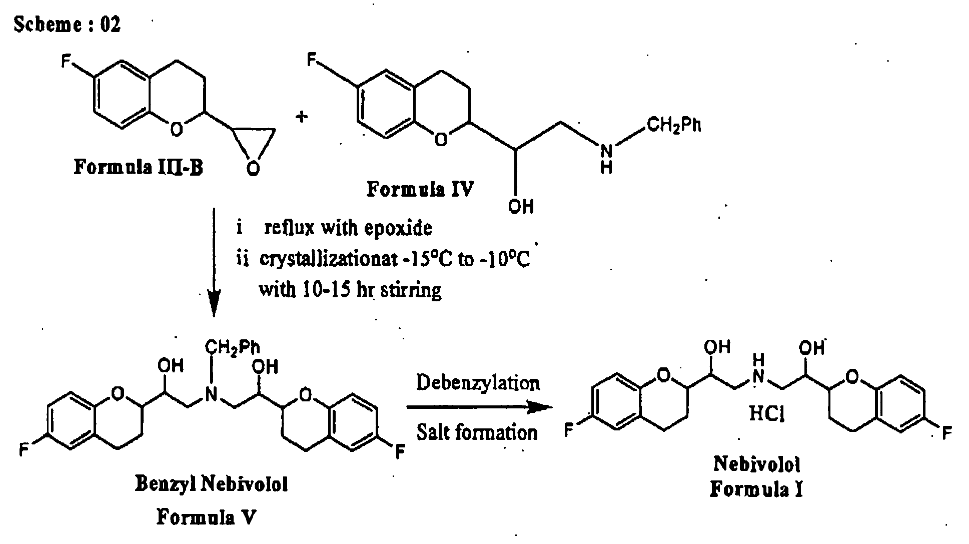

Nabivolol hydrochloride is isolated at -15 to -10 °C and 10-15 hours stirring which increases usage of cryogenic utility and time cycle for commercial production.

Bai Yihui et al. Journal of the Chemical Research, 2006, 807-808 disclose the formation of the base of Nebivolol in a process similar to the previous. Chandrasekhar S. et al. Tetrahedron 2000, 56 (34), 6339-6344 disclose the direct formation of the hydrochloride salt of Nebivolol from an unprotected amine and the oxirane without passing through the benzyl intermediate.

PCT publication WO2008040528 describes a process for the preparation of Nebivolol and, more particularly, to an improved method of synthesizing 6-fluoro chroman, which is key epoxide intermediate of formula (I) for the preparation of Nebivolol.

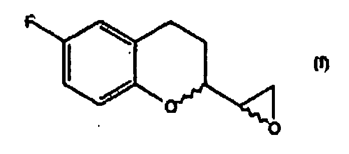

WO2008064826 describes a process for preparing nebivolol and, more particularly, to an improved process for synthesizing enantiomerically enriched 6-fluoro chroman alcohol or epoxide derivatives of formula (I), wherein R and X is defined in the description; as useful intermediates in the preparation of Nebivolol.

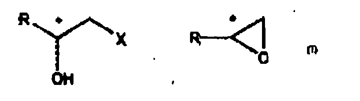

PCT publication WO2008064827 describes a process for preparing nebivolol and, more particularly, to an improved process for synthesizing enantiomerically enriched 6-fluoro chroman alcohol or epoxide derivatives of formula, wherein R and X is defined in the description; as useful intermediates in the preparation of Nebivolol.

U.S. Patent Publication no. 20080221340 discloses a process for the production of the racemic active ingredient nebivolol, in which diastereomeric cyanohydrins are produced, separated, and the separated diastereomers are coupled to one another after a transformation, preferably a partial or complete reduction of the cyano group or a Pinner saponification.

U.S. Patent Publication No. 2007/021623 describes process for preparation of benzyl Nebivolol intermediate and Nebivolol hydrochloride. In the process (±)- [1S*(R*)]-6-fluoro-3,4-dihydro-α-[[(phenylmethyl)amino]methyl]-2H-1- benzo-pyran-2-methanol is treated with (±)-[1S*(S*)]-6-fluoro-3,4-dihydro-2-oxiranyl -2H-1-benzopyran to give (±)-[2R* [1S*, 5S* (S*)]]+[2R* [1S*,5R*(R*)]] α, α'-[(phenylmethyl)imino bis(methylene)]bis[6-Fluoro-3,4 dihydro-]-2H-1-benzopyran-2-methanol, which is then in situ converted to its hydrochloride with HCI gas and crystallized to give desired isomer (±)-[2R* [1S*, 5S* (S*)]] α, α'-[(phenylmethyl)imino bis(methylene)]bis[6-fluoro-3,4dihydro-]-2H-1-benzo pyran-2-methanol. Further, this hydrochloride salt is converted to free base by treatment with inorganic base. Then obtained free base is hydrogenated using 10 % palladium on charcoal to give (±)-[2R* [1S*, 5S* (S*)]] α, α'-[iminobis(methylene)]bis[6-fluoro-3,4dihydro-] -2H-1-benzo pyran-2-methanol. Finally Nebivolol base is converted to Nebivolol hydrochloride by using hydrochloric acid gas in an organic solvent (Scheme III).

There are four steps involved in preparing Nebivolol Hydrochloride. Here purification and isolation of intermediate are required to get desired isomeric pair. Hence the process is lengthy and cost inefficient.

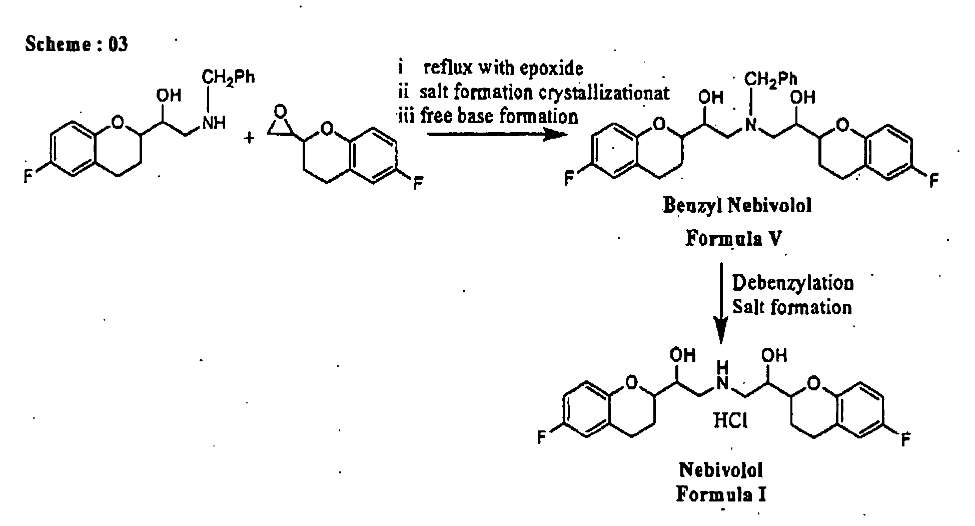

PCT publication WO2007/083318 describes the purification process in ether for benzylated Nebivolol to give the desired isomeric pair of benzylated Nebivolol. Thus, there exists a need for developing a method for the synthesis of the desired diastereomeric mixture of higher purity with the reduced level of undesirable isomeric impurities and avoiding the use of hazardous chemicals, while at the same time reducing number of steps in the synthesis of Nebivolol hydrochloride.

The present invention improves yield of DL-nebivolol hydrochloride by avoiding the steps of crystallization of acid addition salt of N-benzyl Nebivolol and conversion of N-benzyl Nebivolol acid addition salt to free N-benzyl Nebivolol.

### SUMMARY OF THE INVENTION

The objective of the present invention is to provide an improved process for the synthesis of pharmaceutically active 2, 2'-iminobisethanol derivative i.e. 2H-1-benzopyran-2-methanol, α,α'-iminobis (methylene)]bis[ 6-fluoro-3,4-dihydro-,[2R* [R*[R*(S*)]]]] (Nebivolol) hydrohalide salt, more particularly Nebivolol hydrochloride salt, as shown in Figure (I),

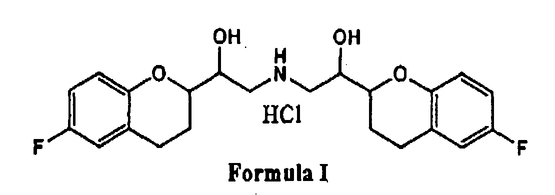

Yet another object of the invention is to provide an improved process for synthesis of Nebivolol hydrochloride salt that involves one pot synthesis starting from (±)-[1S*(R*)] epoxy monomer[ epoxy monomer herein defined as compound-II of schemes IV and V ] without isolation of intermediates.

Yet another object of the invention is to avoid use of column chromatography for purification of N-benzyl Nebivolol.

Yet another object of the invention is to avoid crystallization of acid addition salt of N-benzyl Nebivolol.

Yet another object of the invention is to avoid conversion of N-benzyl Nebivolol acid addition salt to free N-benzyl Nebivolol and its isolation.

### DETAILED DESCRIPTION OF THE INVENTION

The present invention provides an improved process for the preparation of pharmaceutically acceptable salts of DL-Nebivolol, more particularly the OL-Nebivolol hydrochloride of formula- I,

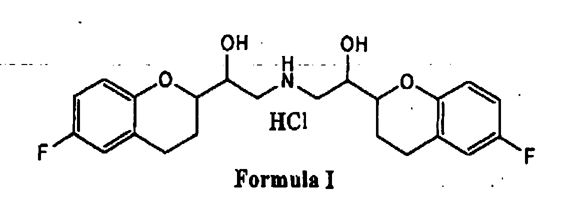

DL-Nebivolol Hydrochloride is prepared according to the present invention by the following route of synthesis,

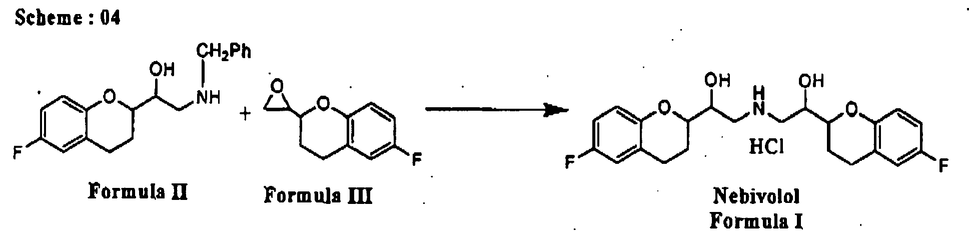

Starting materials, (±)-[16*(S*)]-6-fluoro-3.4-dihydro-2-oxiranyl -2H-1-benzopyran (III) and (±)-[18*(R*)]-6-Fluoro-3,4-dihydro-2-oxiranyl -2H-1-benzopyran (V) are generated by known method, and are separated by column chromatography as per known process disclosed in U. S. Pat. No. 4,654,362.

Another starting material, (±)-[1S*(R*)]-6-ftuoro-3, 4-dihydro-a-[[(phenylmethyl) amino] methyl] -2H-1-benzopyran-2-methanol (II) is generated by the known prior art method described in U. S. Pat. No. 4,654,362, in which (±)-[1S*(R*)]-6-Fluoro-3,4-dihydro-2-oxiranyl -2H-1-benzopyran (V) was reacted with benzylamine (VI) in isopropyl alcohol as shown in scheme-V.

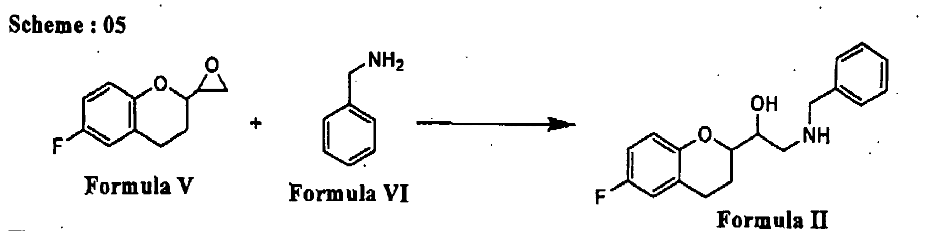

This process comprises the following steps:

### (i) Preparation of N-benzyl Nebivolol:

(±)-[1S*(R*)]-6-fluoro-3, 4-dihydro-α- [[(phenylmethyl) amino] methyl]-2H-1-benzo pyran-2-methanol (III) is reacted with (±)-[1S*(S*)]-6-fluoro-3, 4-dihydro-2-oxiranyl -2H-1-benzopyran (II) in organic solvent. The solvent used for the reaction is selected from alcohols preferably methyl alcohol. The reaction is camed out at 25-70°C, preferably at 65-70° C. and in 8-18 hours, preferably in 16-18 hrs.

### (ii) Debenzylation and preparation of DL-Nebivolol hydrohalide, preferably Nebivolol hydrochloride:

The reaction mass from step (i) is hydrogenated using palladium on charcoal in the presence of aryl halide (VII), preferably benzyl chloride. Using different aryl halides, corresponding hydrohalide salts of DL-nebivolol are obtained.

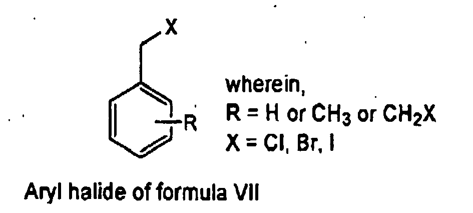

The reaction is carried out at hydrogen pressure of 3kg-6kg preferably at 5-5.5 kg pressure and for 1-5 hrs, preferably 2-3 hrs. The reaction is carried out using 10-15% palladium on carbon preferably at 14-15%. The preferred aryl halide is benzyl chloride The amount of aryl halide used in the reaction is 1.0 to 1.3 moles per mole of epoxy monomer, preferably 1.25 moles per moles of epoxy monomer.

### (iii) Isolation of DL-Nebivolol hydrochloride from reaction mass:

After completion of the reaction, the reaction mass is filtered through hyflo and the filtrate is concentrated to 1/10^{th} of its volume and then cooled to about 33-38°C and stirred for about 5 to 10 minutes The product , DL-nebivolol hydrochloride which separates on cooling is isolated and dried.

Alternatively the step (i) is carried out by reversing the order of starting material epoxy monomer and epoxide, wherein (±)-[1S*(S*)]-6-fluoro-3, 4-dihydro-a- [[(phenylmethyl) amino] methyl]-2H-1-benzo pyran-2-methanol (III) is reacted with (±)-[1S*(R*)]-6-fluoro-3, 4-dihydro-2-oxiranyl -2H-1-benzopyran (II) in organic solvent.

The present invention is illustrated by following non-limiting examples:

### Example-1

### Preparation of (±)-(1S*(R*)]-6-fluoro-3, 4-dihydro-α-[[(phenylmethyl) amino] methyl]-2H-1-benzo pyran-2-methanol (II)

A mixture of benzyl amine (110.3 gm, 1.03mol), (±)-[1S*(R*)]-6-fluarm3,4-dihydro-2. oxiranyl-2H-1-benzo pyran (40 gm, 0.206mol) in 50 ml IPA and 150 ml IPA was stirred at 25-30°C for 4 hours. Then reaction mixture was cooled to 0-5°C and maintained the temperature for 30-45 min. Solid material was precipitated out. The solid was filtered and washed with chilled IPA. Dried to give 50 gm of (±)-[1S*(R*)]-6-fluoro-3, 4-dihydro-α-[[(phenylmethyl) amino] methyl]-2H-1-benzo pyran-2-methanol (II) (HPLC 99.0 %).

### Example-2

### Preparation of Nebivolol hydrochloride

A mixture of (±)-[1S*(S*)]-6-fluoro-3,4-dihydro-2-oxiranyl -2H-1-benzopyran (26.55 gm, 0.136mol)-(III) in 80 ml Methanol, (±)-(1S*(R*)]-6-fluoro-3,4-dihydroα[[(phenylmethyl)amino]methyl]-2H-1-benzo pyran-2-methanol (40 gm, 0.132 mol)-(II) and methanol (120 ml) was stirred at 65-70 °C for 15-18 hours. The reaction mass was diluted with methanol (1000 ml) and added benzyl chloride (21.36 gm, mol), palladium on carbon (10 %, 9.9 gm) to the hydrogenator. Hydrogen pressure was applied approximate 5.0-5.5 Kg/Cm² and heated to 48-52 °C for 2-3 hours. The reaction mass was cooled and filtered to separate the catalyst. 85-90 % of methanol was distilled off at atmospheric pressure and cooled the reaction mass to 35-37°C. Stirred for 5 min, filtered and washed with methanol. The solid was dried to get 26.3 gm Nebivolol Hydrochloride salt (HPLC: 99.5 %)

## Claims

1. An improved process for The preparation of a hydrohalide salt of (±)-[2R*[1S*.5S*(S*)]]-α,α* (iminobis(methylene)]bis[6-fluoro-3,4-dihydro-2H-1-benzopyran-2-methanol] 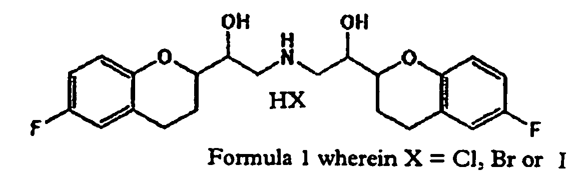 comprising the steps of:
a) Treating (±)-[1S*(R*)]-6-nuoro-3,4-dihydro-α[[(phenytrmeltyl)amino] methyl]-2H-1-benzo pyran-2-methanol (II), with (±)[1S*(S*)-6-fluoro-3,4-dihydro-2-cxiranyt 2H-1-benzopyran (III), in presence of an organic solvent, to give N-protected nebivobt, 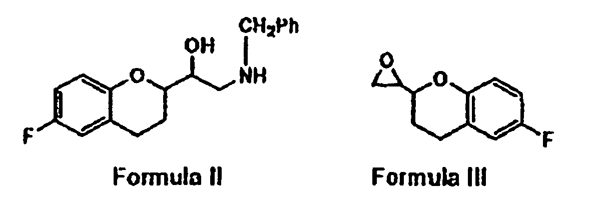
b) deprotecting the compound obtained in (a), by catalytic hydrogenation, in presence of an aryl halide (VII) at elevated temperature and pressure to provide nobivolol acid addition sall. 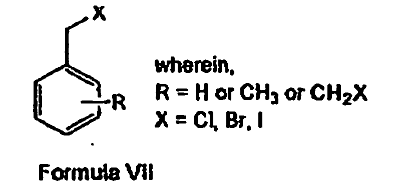
c) Separating the catalyst, concentrating the solution, followed by cooling the reaction mass up to about 30-40°C and separating DL-nebivolol hydrohalide salt.

2. The process according to claim 1, where in the organic solvent in step-a) is selected from C1 to C6 alcohol

3. The process according to claim 2, wherein alcohol is methanol

4. The process according to claim 1, wherein aryl halide in step (b),1s benzyl chloride, benzyl bromide or benzyl iodide.

5. The process of claim-4, wherein aryl halide is benzyl chloride.

6. The process as claimed in claim 1, wherein in isolation of DL-nebivolol hydrohalide salt, in step-(c) is carried out at 35°C to 37°C temperature.

7. The process as claimed in claim-6 wherein DL-nebivolol hydrohalide salt, in step (c) is, DL nobivolol hydrochloride.

8. The process as claimed in claim 7, wherein in the nebivolol hydrochloride obtained is a mixture of RSSS and SRRR isomers.

9. The process as claimed in claim 7, wherein in the purity of the DL-nebivolol hydrochloride is more than 99.5 %.

10. The process according to claim 1, wherein said hydrohalide salt is the hydrochloride salt.

## Patentansprüche

1. Verbessertes Verfahren für die Herstellung eines Hydrohalogenidsalzes von (±)-[2R*[1S*,5S*(S*))]-α,α'-[Iminobis(methylen)]bis[6-fluor-3,4-dihydro-2H-1-benzo-pyran-2-methanol] 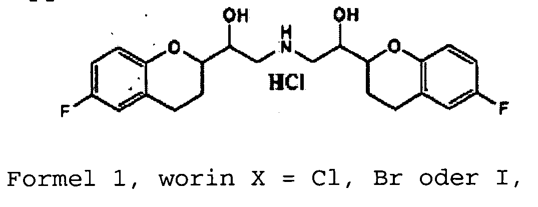 umfassend die folgenden Schritte:
a) Behandeln von (±)-{1S*(R*)}-6-Fluor-3,4-dihydro-α[[(phenylmethyl)amino]-methyl}-2H-1-benzo-pyran-2-methanol (II) mit (±)-{1S*(S*)]-6-Fluor-3,4-dihydro-2-oxiranyl-2H-1-benzopyran (III) in Gegenwart eines organischen Lösungsmittels unter Erhalt von N-geschütztem Nebivolol, 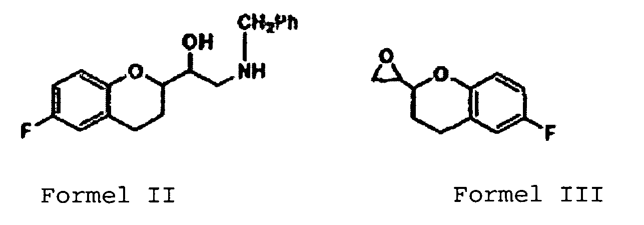
b) Entschützen der in (a) erhaltenen Verbindung durch katalytische Hydrierung in Gegenwart eines Arylhalogenids (VII) bei erhöhter Temperatur und Druck zur Bereitstellung eines Nebivolol-Säureadditionssalzes, 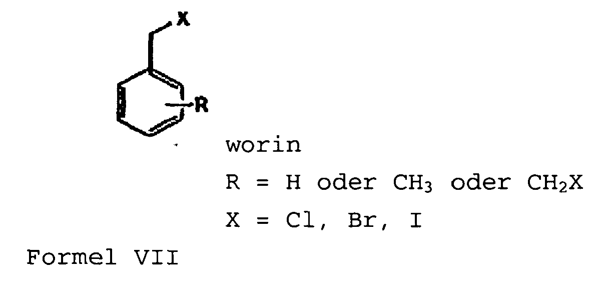
c) Abtrennen des Katalysators, Konzentrieren der Lösung, gefolgt von einem Kühlen der Reaktionsmasse auf bis zu etwa 30 - 40°C und Abtrennen von DL-Nebivolol-Hydrohalogenidsalz.

2. Verfahren gemäß Anspruch 1, wobei das organische Lösungsmittel in Schritt a) aus C1- bis C6-Alkohol gewählt ist.

3. Verfahren gemäß Anspruch 2, wobei Alkohol Methanol ist.

4. Verfahren gemäß Anspruch 1, wobei Arylhalogenid in Schritt (b) Benzylchlorid, Benzylbromid oder Benzyliodid ist.

5. Verfahren gemäß Anspruch 4, wobei Arylhalogenid Benzylchlorid ist.

6. Verfahren gemäß Anspruch 1, wobei eine Isolierung von DL-Nebivolol-Hydrohalogenidsalz in Schritt (c) bei einer Temperatur von 35°C bis 37°C durchgeführt wird.

7. Verfahren gemäß Anspruch 6, wobei das DL-Nebivolol-Hydrohalogenidsalz in Schritt (c) DL-Nebivolol-Hydrochlorid ist.

8. Verfahren gemäß Anspruch 7, wobei das erhaltene Nebivolol-Hydrochlorid eine Mischung von RSSS- und SRRR-Isomeren ist.

9. Verfahren gemäß Anspruch 7, wobei die Reinheit des DL-Nebivolol-Hydrochlorids mehr als 99,5 % beträgt.

10. Verfahren gemäß Anspruch 1, wobei das Hydrohalogenidsalz das Hydrochloridsalz ist.

## Revendications

1. Procédé amélioré de préparation d'un sel d'halogénohydrate de (±)-[2R*[1S*,5S*(S*)]]-α,α'-[iminobis(méthylène)]bis[6-fluoro-3,4-dihydro-2H-1-benzopyrane-2-méthanol] 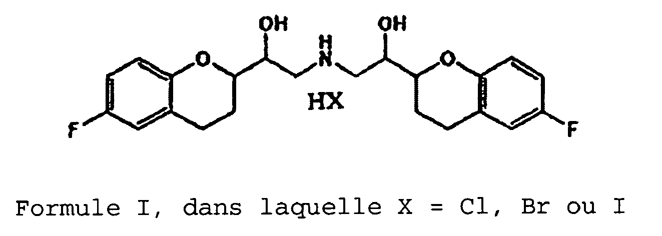 comprenant les étapes suivantes :
a) le traitement de (±)-[1S*(R*)]-6-fluoro-3,4-dihydro-α[[(phénylméthyl)amino]méthyl]-2H-1-benzopyrane-2-méthanol (II) avec du (±)-[1S*(S*)]-6-fluoro-3,4-dihydro-2-oxiranyl-2H-1-benzopyrane (III) en présence d'un solvant organique, pour obtenir du nébivolol N-protégé, 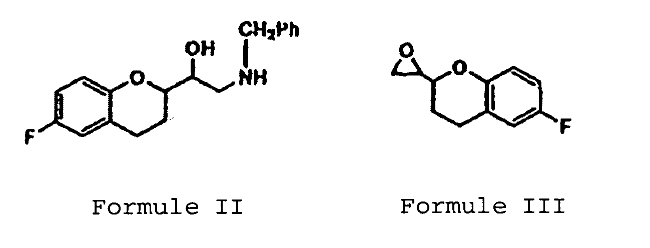
b) la déprotection du composé obtenu en (a), par hydrogénation catalytique, en présence d'un halogénure d'aryle (VII) à température et pression élevées pour obtenir un sel d'addition acide de nébivolol, 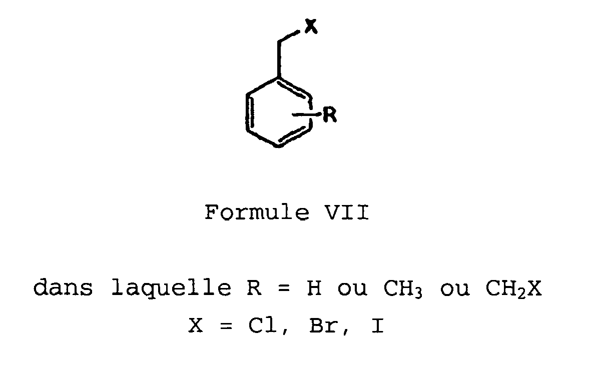
c) la séparation du catalyseur, la concentration de la solution, suivie par le refroidissement de la masse réactionnelle jusqu'à environ 30 à 40 °C et la séparation du sel d'halogénohydrate de DL-nébivolol.

2. Procédé selon la revendication 1, dans lequel le solvant organique à l'étape a) est choisi parmi les alcools en C1 à C6.

3. Procédé selon la revendication 2, dans lequel l'alcool est le méthanol.

4. Procédé selon la revendication 1, dans lequel l'halogénure d'aryle à l'étape (b) est le chlorure de benzyle, le bromure de benzyle ou l'iodure de benzyle.

5. Procédé selon la revendication 4, dans lequel l'halogénure d'aryle est le chlorure de benzyle.

6. Procédé selon la revendication 1, dans lequel l'isolement du sel d'halogénohydrate de DL-nébivolol à l'étape (c) est réalisé à une température de 35 °C à 37 °C.

7. Procédé selon la revendication 6, dans lequel le sel d'halogénohydrate de DL-nébivolol à l'étape (c) est le chlorhydrate de DL-nébivolol.

8. Procédé selon la revendication 7, dans lequel le chlorhydrate de nébivolol obtenu est un mélange des isomères RSSS et SRRR.

9. Procédé selon la revendication 7, dans lequel la pureté du chlorhydrate de DL-nébivolol est supérieure à 99,5 %.

10. Procédé selon la revendication 1, dans lequel le sel d'halogénohydrate est le sel de chlorhydrate.
